# EUROPEAN PATENT SPECIFICATION

(11) **EP 2 480 245 B1**
(45) Date of publication and mention of the grant of the patent: **07.05.2014**
(21) Application number: 10754768.9
(22) Date of filing: 22.09.2010
(51) Int. Cl.: C07K 14/47, C12P 21/06, A61K 38/17, A61K 38/48

(54) **A NOVEL METHOD FOR THE PRODUCTION OF A ANTIMICROBIAL PEPTIDE**
Neuartiges Verfahren zur Herstellung von antimikrobiellen Peptiden
Nouveau procédé de production d'un peptide antimicrobien

(30) Priority: 25.09.2009 EP 09012228
(43) Date of publication of application: 01.08.2012
(73) Proprietor: B.R.A.I.N. Biotechnology Research And Information Network AG, 64673 Zwingenberg (DE)
(72) Inventor: NAUMER, Christian, 67459 Böhl-Iggelheim (DE); KROHN, Michael, 64653 Lorsch (DE); TIFFERT, Yvonne, 69161 Mannheim (DE); ECK, Jürgen, 64625 Bensheim-Auerbach (DE)
(74) Representative: Vossius & Partner
(86) International application number: PCT/EP2010/063971
(87) International publication number: WO 2011/036174

(56) References cited:
- WO-A-02/100895
- WO-A1-2005/106039
- WO-A2-2008/030988
- SCHITTEK B ET AL: "DERMICIDIN: A NOVEL HUMAN ANTIBIOTIC PEPTIDE SECRETED BY SWEAT GLANDS", NATURE IMMUNOLOGY, NATURE PUBLISHING GROUP, GB, vol. 2, no. 12, 1 December 2001 (2001-12-01), pages 1133-1137, XP001094755, ISSN: 1529-2908, DOI: DOI:10.1038/NI732
- CIPAKOVA I ET AL: "Expression and purification of human antimicrobial peptide, dermcidin, in Escherichia coli", PROTEIN EXPRESSION AND PURIFICATION, ACADEMIC PRESS, SAN DIEGO, CA, vol. 45, no. 2, 1 February 2006 (2006-02-01), pages 269-274, XP024908621, ISSN: 1046-5928, DOI: DOI:10.1016/J.PEP.2005.07.002 [retrieved on 2006-02-01]
- LAI Y-P ET AL: "Functional and structural characterization of recombinant dermcidin-1L, a human antimicrobial peptide", BIOCHEMICAL AND BIOPHYSICAL RESEARCH COMMUNICATIONS, ACADEMIC PRESS INC. ORLANDO, FL, US, vol. 328, no. 1, 4 March 2005 (2005-03-04) , pages 243-250, XP004723765, ISSN: 0006-291X, DOI: DOI:10.1016/J.BBRC.2004.12.143

## Description

The present invention is defined by the claims. The present invention relates to a method of producing a peptide consisting of the amino acids 63 to 110 of dermcidin (SEQ ID NO: 3) without sequences extending beyond said peptide comprising (a) culturing a host cell carrying a nucleic acid molecule encoding the peptide without sequences extending beyond the peptide in an expressible form, and (b) optionally isolating the peptide from the culture, wherein the host cell is a prokaryote cell or a fungal/yeast cell.

Although the skin provides a remarkably good barrier against bacterial infections (e.g. acid mantel and antimicrobial peptides of the skin) microbial skin infections do occur, frequently. They can range in size from a tiny spot to the entire body surface, and can range in seriousness as well, from harmless to life threatening. Many types of bacteria and fungi can infect the skin. The most common are of the bacteria genera *Staphylococcus* and *Streptococcus.* Some people are at particular risk of contracting skin infections. For example, people with diabetes are likely to have poor blood flow, especially to the hands and feet, and the high levels of sugar in their blood decrease the ability of white blood cells to fight infections. People having a weakened immune system, like people with human immunodeficiency virus (HIV)/AIDS or other immune disorders, or people undergoing chemotherapy, are at higher risk as well. Skin that is inflamed or damaged by sunburn, scratching, or other trauma is more likely to be infected. In fact, any break in the skin predisposes a person to infection. In order to treat or prevent microbial skin infections, antimicrobial creams and ointments may be applied to open areas to keep the tissue moist and to try to prevent bacterial invasion. If an infection develops, small areas may be treated with antimicrobial creams. Larger areas require antimicrobials taken orally or administered by injection. A disadvantage of the continuous use of antimicrobials (such as antibiotics) is that their use has led to the development of multi-resistant bacterial strains [1].

Although the skin is permanently exposed to microorganisms, it is normally not infected. Among others antimicrobial peptides, which are numerous on the epidermis, are an explanation for this phenomenon. They control microbial growth, in particular while the skin is damaged and during the healing process. In general, antimicrobial peptides are endogenous, gene-encoded peptides with an important role in the early phase of pathogen defence. Examples of antimicrobial peptides of the skin are cathelicidins, β-defensins and dermcidin (DCD). Catelecidine and β-defensins are primarily expressed in response to wounding of the skin and do not participate in the constant modulation of the epithelial defence mechanism [2]. Moreover, most antimicrobially active peptides are not active against all know microbial pathogens. Defensins for example do not affect *S*. *aureus.*

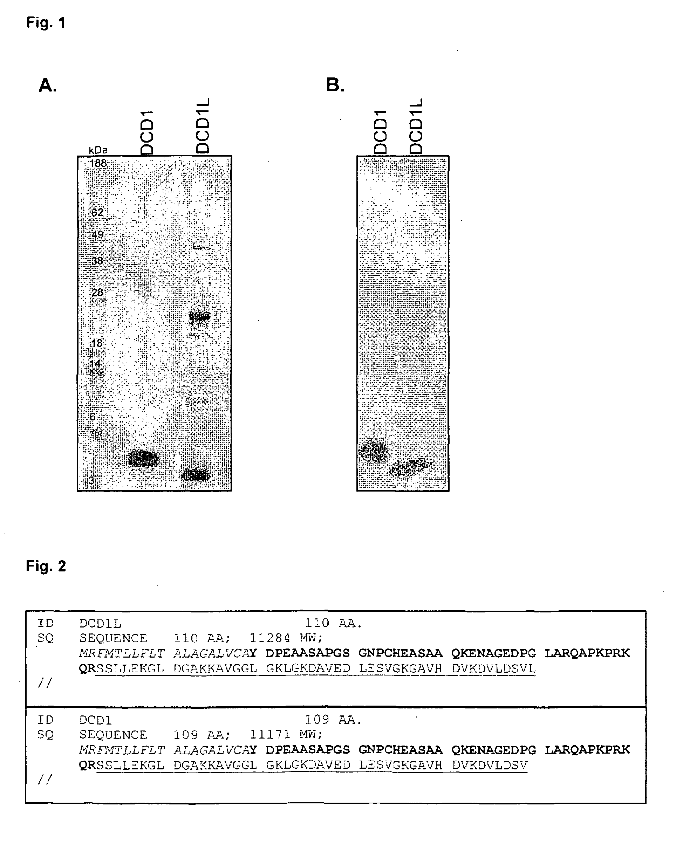

This is in contrast to dermcidin which is constitutively expressed in eccrine sweat glands and secreted via sweat to the epidermal surface and is antimicrobially active against a number of skin infective bacteria, including *Staphylococci* and *Streptococci* [3, 4]. Many antibacterial peptides exert their effect by altering the permeability of the inner or outer membrane in gram-negative bacteria. In contrast to this, DCD1L and derived peptides do not show this behaviour [6]. The mode of action of the antimicrobially active peptides is so far unknown [3, 6]. Dermcidin is composed of 110 amino acids. It is proteolytically processed to form several peptides with antimicrobial activity. The initial cleavage at R62 (Arg 62, arginine 62) is effected by a so far unknown Arg-specific protease [5]. The active peptides do not contain Arg residues (Fig 2, underlined and/or bold sequences) and are thus not susceptible to degradation by Arg-specific proteases. Further processing to yield shorter peptides, most of which retain antimicrobial activity, is done by several endoproteases and carboxypeptidases, for example cathepsin D [5]. Different DCD derived peptides demonstrate different spectra of activity [6]. Moreover, these peptides show antimicrobial activity against several microorganisms like *E. coli,* methicillin-resistant *Staphylococcus aureus, Staphylococcus epidermidis* and *Candida albicans.* Different DCD derived peptides demonstrate different spectra of activity [6].

European Patent EP 1397384 B1 describes the original isolation of dermcidin. The European patent EP 1397384 B1 claims antimicrobial peptides comprising a fragment of a maximum of 50aa, derived from the C-terminus of dermcidin. In example 3, the construction of a fusion protein encoded by the complete dermcidin cDNA (encoding 110aa) and 3' thereof the eGFP gene is described. Attempts to cleave the fusion protein with an arginine-specific endoprotease failed, although the fusion protein contained arginine residues in positions 53, 59 and 62 of the mature protein. Accordingly, EP 1 397 384 B1 in Example 4 only refers to a antimicrobial fusion protein encoded by a gene encoding aa 47 to 110 of dermcidin and 5' thereof the eGFP gene. The authors concluded that there is no cleavage site for this arginine-specific protease in the fusion protein.

Therefore, subsequent attempts for the expression of genes encoding dermcidin fusion proteins relied on different strategies to cleave off an N-terminal fusion partner of dermcidin. For example, Lai et al., BBRC 328 (2005), 243-250 [7] describe the recombinant construction of a fusion protein containing an N-terminal thioredoxin and a histidine tag for purification. In addition, the fusion protein contained a Factor Xa cleavage site. Upon recombinant expression of the fusion protein, this was purified via the His-tag and cleaved by Factor Xa. The approach taken by Lai et al. has the disadvantage that a Factor Xa cleavage site has to be recombinantely engineered into the plasmid. In addition, Factor Xa is costly which precludes its use in large scale production. A different strategy for producing dermcidin as a fusion protein was employed by 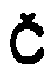 ipáková et al. [8] who inserted the sequence encoding the 47 C-terminal amino acids of dermcidin between the ketosteroid isomerase gene and the His₆Tag sequence. In addition, the dermcidin sequence was flanked by triplets encoding methionine. Recombinant production led to the formation of the desired fusion proteins in the form of inclusion bodies which were further processed and finally cleaved by CNBr action. This protocol has the drawback that the final product is only obtainable after time-consuming processing of bacterial inclusion bodies.

In this context there is an ongoing demand for novel antimicrobially active compounds and methods for producing these compounds. This need is addressed by the present invention.

Accordingly, the present invention relates to a method of producing a peptide consisting of the amino acids 63 to 110 of dermcidin (SEQ ID NO: 3) without sequences extending beyond said peptide comprising
(a) culturing a host cell carrying a nucleic acid molecule encoding the peptide without sequences extending beyond said peptide in an expressible form, and
(b) optionally isolating the peptide from the culture, wherein the host is a prokaryote cell is a prokaryote cell or a fungal/yeast cell.

The term "peptide" as used herein describes linear molecular chains of amino acids, including single chain molecules or their fragments, containing preferably at least 23 amino acids. In connection with this invention the term "peptide" bears the same meaning as the term "polypeptide" and these terms may be interchangeably used, if the molecule contains at least preferably 23 amino acids. In other words, the term polypeptide preferably does not pertain to amino acid molecules of less than 23 amino acids. In particular peptides used as tags may comprise less than 23 amino acids. Peptides may further form oligomers consisting of at least two identical or different molecules. The corresponding higher order structures of such multimers are, correspondingly, termed homo- or heterodimers, homo- or heterotrimers etc. Furthermore, peptidomimetics of such peptides where amino acid(s) and/or peptide bond(s) have been replaced by functional analogs are also encompassed by the invention. Such functional analogues include all known amino acids other than the 20 gene-encoded amino acids, such as selenocysteine. The term "peptide" also refers to naturally modified peptides where the modification is effected e.g. by glycosylation, acetylation, phosphorylation and similar modifications which are well known in the art.

The term "nucleic acid molecule", in accordance with the present invention, includes DNA, such as cDNA or genomic DNA, and RNA. It is understood that the term "RNA" as used herein comprises all forms of RNA including mRNA. Preferably the term "nucleic acid molecule" is genomic DNA, cDNA or mRNA. The nucleic acid sequence may also comprise regulatory regions or other untranslated regions. The term "nucleic acid molecule" is interchangeably used in accordance with the invention with the term "polynucleotide". Further included are nucleic acid mimicking molecules known in the art such as synthetic or semisynthetic derivatives of DNA or RNA and mixed polymers, both sense and antisense strands. They may contain additional non-natural or derivatized nucleotide bases, as will be readily appreciated by those skilled in the art. In a preferred embodiment the polynucleotide or the nucleic acid molecule(s) is/are DNA. Such nucleic acid mimicking molecules or nucleic acid derivatives according to the invention include phosphorothioate nucleic acid, phosphoramidate nucleic acid, 2'-O-methoxyethyl ribonucleic acid, morpholino nucleic acid, hexitol nucleic acid (HNA) and locked nucleic acid (LNA) (see, for example, Braasch and Corey, Chemistry & Biology 8, 1-7 (2001)). LNA is an RNA derivative in which the ribose ring is constrained by a methylene linkage between the 2'-oxygen and the 4'-carbon.

For the purposes of the present invention, a peptide nucleic acid (PNA) is a polyamide type of DNA analog. The monomeric units for the corresponding derivatives of adenine, guanine, thymine and cytosine are available commercially (for example from Perceptive Biosystems). PNA is a synthetic DNA-mimic with an amide backbone in place of the sugar-phosphate backbone of DNA or RNA. As a consequence, certain components of DNA, such as phosphorus, phosphorus oxides, or deoxyribose derivatives, are not present in PNAs. As disclosed by Nielsen et al., Science 254:1497 (1991); and Egholm et al., Nature 365:666 (1993), PNAs bind specifically and tightly to complementary DNA strands and are not degraded by nucleases. Furthermore, they are stable under acidic conditions and resistant to proteases (Demidov et al. (1994), Biochem. Pharmacol., 48, 1310-1313). Their electrostatically neutral backbone increases the binding strength to complementary DNA as compared to the stability of the corresponding DNA-DNA duplex (Wittung et al. (1994), Nature 368, 561-563; Ray and Norden (2000), Faseb J., 14, 1041-1060). In fact, PNA binds more strongly to DNA than DNA itself does. This is probably because there is no electrostatic repulsion between the two strands, and also the polyamide backbone is more flexible. Because of this, PNA/DNA duplexes bind under a wider range of stringency conditions than DNA/DNA duplexes, making it easier to perform multiplex hybridization. Smaller probes can be used than with DNA due to the strong binding. In addition, it is more likely that single base mismatches can be determined with PNA/DNA hybridization because a single mismatch in a PNA/DNA 15-mer lowers the melting point (Tₘ) by 8°-20° C, vs. 4°-16° C for the DNA/DNA 15-mer duplex. Thereby discrimination between perfect matches and mismatches is improved. For its uncharged nature, PNA also permits the hybridisation of DNA samples at low salt or no-salt conditions, since no inter-strand repulsion as between two negatively charged DNA strands needs to be counteracted. As a consequence, the target DNA has fewer secondary structures under hybridisation conditions and is more accessible to probe molecules.

The "host cell" in accordance with the invention may be produced by introducing the nucleic acid molecule or vector(s) of the invention (described herein below) into the host cell which upon its/their presence mediates the expression of the nucleic acid molecule described herein encoding the peptide or fusion protein (see below) described herein. The host from which the host cell is derived may be any prokaryote or a fungal/yeast cell. The fungal/yeast cell may be a *Saccharomyces cerevisiae* cell, *Pichia pastoris* cell or an *Aspergillus* cell.

Suitable prokaryotes (bacteria) useful as hosts for the invention are those generally used for cloning and/or expression like *E. coli* (e.g., *E coli* strains BL21, HB101, DH5a, XL1 Blue, Y1090 and JM101), *Salmonella typhimurium, Serratia marcescens, Burkholderia glumae, Pseudomonas putida, Pseudomonas fluorescens, Pseudomonas stutzeri, Streptomyces lividans, Lactococcus lactis, Mycobacterium smegmatis, Streptomyces* or *Bacillus subtilis.* Appropriate culture mediums and conditions for the above-described host cells are known in the art.

Preferred examples for host cell to be genetically engineered with the nucleic acid molecule or the vector(s) of the invention is a cell of yeast, *E*. *coli* and/or a species of the genus *Bacillus* (e.g., *B. subtilis*). Most preferred the host cell is a yeast cell (e.g. *S. cerevisiae*).

The term "vector" in accordance with the invention means preferably a plasmid, cosmid, virus, bacteriophage or another vector used e.g. conventionally in genetic engineering which carries the nucleic acid molecule of the invention either encoding the peptide or the fusion protein of the invention. Accordingly, the nucleic acid molecule of the invention may be inserted into several commercially available vectors. Non-limiting examples include prokaryotic plasmid vectors, such as of the pUC-series, pBluescript (Stratagene), the pET-series of expression vectors (Novagen) or pCRTOPO (Invitrogen) and vectors compatible with an expression in mammalian cells like pREP (Invitrogen), pcDNA3 (Invitrogen), pCEP4 (Invitrogen), pMC1neo (Stratagene), pXT1 (Stratagene), pSG5 (Stratagene), EBO-pSV2neo, pBPV-1, pdBPVMMTneo, pRSVgpt, pRSVneo, pSV2-dhfr, pIZD35, pLXIN, pSIR (Clontech), pIRES-EGFP (Clontech), pEAK-10 (Edge Biosystems) pTriEx-Hygro (Novagen) and pCINeo (Promega). Examples for plasmid vectors suitable for *Pichia pastoris* comprise e.g. the plasmids pAO815, pPIC9K and pPIC3.5K (all Intvitrogen).

The nucleic acid molecule referred to herein may be inserted into vectors such that a translational fusion with another polynucleotide is generated. The other polynucleotide may encode a peptide heterologous with regard to dermcidin (e.g. a peptide-tag) as described herein below, which may e.g. increase the solubility and/or facilitate the purification of the fusion protein. The vectors may also contain an additional expressible polynucleotide coding for one or more chaperones to facilitate correct protein folding. For vector modification techniques, see Sambrook and Russel (2001), Cold Spring Harbor Laboratory; 3rd edition. Generally, vectors can contain one or more origin of replication (ori) and inheritance systems for cloning or expression, one or more markers for selection in the host, e. g., antibiotic resistance, and one or more expression cassettes. Suitable origins of replication (ori) include, for example, the Col E1, the SV40 viral and the M 13 origins of replication.

The coding sequences inserted in the vector can e.g. be synthesized by standard methods, or isolated from natural sources. Ligation of the coding sequences to transcriptional regulatory elements and/or to other amino acid encoding sequences can be carried out using established methods. Transcriptional regulatory elements (parts of an expression cassette) ensuring expression in prokaryotes or eukaryotic cells are well known to those skilled in the art. These elements comprise regulatory sequences ensuring the initiation of transcription (e. g., translation initiation codon, promoters, such as naturally-associated or heterologous promoters and/or insulators), internal ribosomal entry sites (IRES) (Owens, Proc. Natl. Acad. Sci. USA 98 (2001), 1471-1476) and optionally poly-A signals ensuring termination of transcription and stabilization of the transcript. Additional regulatory elements may include transcriptional as well as translational enhancers. Preferably, the polynucleotide encoding the peptide or fusion protein described herein is operatively linked to such expression control sequences allowing expression in prokaryotes or eukaryotic cells. The vector may further comprise nucleotide sequences encoding secretion signals as further regulatory elements. Such sequences are well known to the person skilled in the art. Furthermore, depending on the expression system used, leader sequences capable of directing the expressed polypeptide to a cellular compartment may be added to the coding sequence of the polynucleotide described herein. Such leader sequences are well known in the art. Furthermore, it is preferred that the vector comprises a selectable marker. Examples of selectable markers include neomycin, ampicillin, and hygromycine, kanamycin resistance and the like. Specifically-designed vectors allow the shuttling of DNA between different hosts, such as bacteria-fungal cells or bacteria-animal cells (e. g. the Gateway® system available at Invitrogen). An expression vector described herein is capable of directing the replication, and the expression, of the polynucleotide and encoded peptide or fusion protein described herein. Apart from introduction via vectors such as phage vectors or viral vectors (e.g. adenoviral, retroviral), the nucleic acid molecules as described herein above may be designed for direct introduction or for introduction via liposomes into a cell. Additionally, baculoviral systems or systems based on vaccinia virus or Semliki Forest virus can be used as eukaryotic expression systems for the nucleic acid molecules of the invention.

A typical mammalian expression vector contains the promoter element, which mediates the initiation of transcription of mRNA, the protein coding sequence, and signals required for the termination of transcription and polyadenylation of the transcript. Moreover, elements such as origin of replication, drug resistance gene, regulators (as part of an inducible promoter) may also be included. The *lac* promoter is a typical inducible promoter, useful for prokaryotic cells, which can be induced using the lactose analogue *isopropylthiol-b-D-galactoside.* ("IPTG"). For recombinant expression and secretion, the polynucleotide of interest may be ligated between e.g. the PeIB leader signal, which directs the recombinant protein in the periplasm and the gene III in a phagemid called pHEN4 (described in Ghahroudi et al, 1997, FEBS Letters 414:521-526). Additional optional elements include enhancers, Kozak sequences and intervening sequences flanked by donor and acceptor sites for RNA splicing. Highly efficient transcription can be achieved with the early and late promoters from SV40, the long terminal repeats (LTRs) from retroviruses, e.g., RSV, HTLVI, HIVI, and the early promoter of the cytomegalovirus (CMV). However, cellular elements can also be used (e.g., the human actin promoter). Alternatively, the recombinant polypeptide can be expressed in stable cell lines that contain the gene construct integrated into a chromosome. The co-transfection with a selectable marker such as dhfr, gpt, neomycin, hygromycin allows the identification and isolation of the transfected cells. The transfected nucleic acid can also be amplified to express large amounts of the encoded polypeptide. As indicated above, the expression vectors will preferably include at least one selectable marker. Such markers include dihydrofolate reductase, G418 or neomycin resistance for eukaryotic cell culture and tetracycline, kanamycin or ampicillin resistance genes for culturing in *E. coli* and other bacteria.

The term "culturing" specifies the process by which host cells are grown under controlled conditions. These conditions may vary dependent on the host cell used. The skilled person is well aware of methods for establishing optimized culturing conditions. Moreover, methods for establishing, maintaining and manipulating a cell culture have been extensively described in the state of the art.

The term "expressible form" in accordance with the invention, means that in the host cell the process can be induced by which information from a nucleic acid molecule encoding the peptide is used in the synthesis of the peptide described herein. Several steps in this process may be modulated, including the transcription, RNA splicing, translation, and post-translational modification of the peptide of the invention by methods know in the art. Accordingly, such modulation may allow for control of the timing, location, and amount of peptide produced.

The term "isolating the peptide" in accordance with the invention refers to a series of methods intended to isolate a single type of peptide or, less preferred, a group of desired peptides from a complex mixture. Suitable methods for isolating peptides from a host cell are well known to the skilled person. The various steps in the isolation method may free the peptide from a matrix that confines it, separate the peptide and non-peptide parts of the mixture, and finally separate the desired peptide from all other peptides. Isolation steps exploit differences in peptide size, physico-chemical properties and binding affinity. In this regard it is preferred that the peptide is exported to the culture medium. Depending on the vector construction employed, the peptide may be exported to the culture medium or maintained within the host cell. Suitable protocols for obtaining the peptide produced are well-known in the art for both ways of peptide production.

Contrary to the accepted published data [7, 8], the inventors have surprisingly found that the peptide represented by amino acids 63 to 110 of dermcidin (SEQ ID NO: 3) does not have antimicrobial activity. Only the peptide represented by amino acids 63 to 109 of dermcidin (SEQ ID NO: 2) was found to have antimicrobial activity. Thus, it is not necessary to produce the peptide consisting of amino acids 63 to 110 of dermcidin (SEQ ID NO: 3) fused to a fusion partner in order to circumvent toxicity for the host cell as described in [7]. According to the method of the invention described herein above, the non-toxic peptide encoded by the amino acids 63 to 110 of dermcidin (SEQ ID NO: 3) can be produced in a host cell directly and without additional sequences extending beyond amino acids 63 to 110 of dermcidin (SEQ ID NO: 3), which facilitates *inter alia* large scale production. Accordingly, it is understood that the nucleic acid molecule according to the main embodiment encodes the peptide directly and without additional sequences extending beyond amino acids 63 to 110 of dermcidin (SEQ ID NO: 3). This peptide can further be activated to have antimicrobial activity by proteolytic cleavage with a Carboxypeptidase as described herein below.

In a preferred embodiment of the invention at least 50 milligrams peptide per litre of cultured host cells are produced.

In the prior art, expression of amounts of at least 50mg/litre of a antimicrobially active derivative of dermcidin were not achieved due to the autotoxicity to the host cells. With increasing preference expression of amounts of at least 50mg/l, at least 100 mg/l, at least 250 mg/l, at least 500mg/l, at least 750mg/l, and at least 1000mg/l are produced. Moreover the envisaged amounts are produced with increasing preference within any time, 72h, 48h, 24h, 12h and 6h. The skilled person is well aware that depending on the host cell culturing conditions and/or culturing time for the production of a least 50 milligrams per litre of cultured cells may be optimized. Means and methods for optimizing culturing conditions and/or culturing time to a host cells are well know in the state of the art. Accordingly, the host cells of the invention are cultured under conditions suitable for the respective microorganism.

In another preferred embodiment of the invention the methods described herein above further comprise the steps of
(c) subjecting the peptide of item 1(b) to proteolytic cleavage with an carboxypeptidase which cleaves off the C-terminal leucine in amino acid position 110 of dermcidin, and
(d) isolating the peptide from the resulting cleavage product.

The term "carboxypeptidase" (EC 3.4.16 - 3.4.18) in accordance with the present invention refers to a an enzyme that hydrolyzes the carboxy-terminal (C-terminal) end of a peptide bond. It is preferred that the carboxypeptidase of the invention cleaves off the C-terminal leucine in amino acid position 110 of dermcidin (SEQ ID No. 1).

The term "proteolytic cleavage" as used herein means the hydrolysis of a peptide bond.

Also described herein is a nucleic acid molecule encoding a fusion protein comprising or consisting of
(a) a peptide heterologous to dermcidin; and, C-terminally thereof
(b) a peptide having the antimicrobial activity of dermcidin
wherein the fusion protein contains an arginine residue located immediately N-terminally of the peptide of (b).

The term "fusion protein" as used herein, is a construct created through the joining of a polynucleotide encoding a peptide having the antimicrobial activity of dermcidin to a polynucleotide encoding a peptide heterologous thereto which may be a tag. Translation of this fusion polynucleotide results in the fusion protein which is a single polypeptide with functional properties derived from dermcidin and the peptide heterologous thereto. It is preferred that the fusion protein is a soluble fusion protein and/or secreted from the host cell. Recombinant fusion proteins are created artificially by recombinant DNA technology well known in the art and may be used in biological research or therapeutics.

The term "peptide heterologous to dermcidin" as used herein refers to an amino acid sequence genetically grafted onto a recombinant peptide, in the present case the peptide having dermcidin activity. Such way a heterologous fusion protein as described herein is preferably experimentally generated and not normally produced (expressed) by a cell. These peptides, preferably tags may be removable by chemical agents or by enzymatic means, such as proteolysis or intein splicing. Tags are attached to proteins for various purposes. Affinity tags are appended to proteins so that they can be purified from their crude biological source using an affinity technique. These include but are not limited to chitin binding protein (CBP), maltose binding protein (MBP), glutathione-S-transferase (GST), and poly(His) tag. The poly(His) tag is a widely-used protein tag; it binds to metal matrices. Solubilization tags are used, especially for recombinant proteins expressed in chaperone-deficient species such as *E. coli,* to assist in the proper folding in proteins and keep them from precipitating. These include but are not limited to thioredoxin (TRX) and poly(NANP). Some affinity tags have a dual role as a solubilization agent, such as MBP, and GST. Chromatography tags are used to alter chromatographic properties of the protein to afford different resolution across a particular separation technique. Chromatography tags comprise but are not limited to of polyanionic amino acids, such as FLAG-tag. Epitope tags are short peptide sequences which are chosen because high-affinity antibodies can be reliably produced in many different species. These are usually derived from viral genes, which explain their high immunoreactivity. Epitope tags include but are not limited to V5-tag, c-myc-tag, and HA-tag. These tags are particularly useful for western blotting and immunoprecipitation experiments, although they also find use in antibody purification. Fluorescence tags are used to give visual readout on a protein. For example, GFP and its variants are the most commonly used fluorescence tags. More advanced applications of GFP include using it as a folding reporter (fluorescent if folded, colorless if not). Moreover, tags find many other usages, such as specific enzymatic modification (such as biotin ligase tags) and chemical modification (FIAsH) tag. Examples of suitable tags to be used in accordance with the invention comprise but are not limited to lacZ, GST, maltose-binding protein, NusA, BCCP, c-myc, CaM, His, FLAG, GFP, YFP, cherry, thioredoxin, poly(NANP), V5, Snap, HA, chitin-binding protein, Softag 1, Softag 3, Strep, or S-protein, and furthermore tags comprising a binding domain capable of binding, directly or indirectly, to the skin.

In this connection, the term a "binding domain capable of binding, directly or indirectly to the skin" specifies a binding domain which binds to an epitope being present directly on the skin or an epitope being present on dermal appendages like hair, glands or nails. In this regard, an epitope has to be understood as the part of a macromolecule (e.g. collagen, keratin, polysaccharides or polysaccharide derivatives, melamine-type polymers, or polyurea) that binds to the binding domain according to the invention. Such binding domains may bind to collagen, keratin, polysaccharides or polysaccharide derivatives, to melamine-type polymers, or to polyurea. Preferably the binding domain is a collagen binding domain, a keratin binding domain, a cellulose binding domain (CBD), a melamine binding domain or a polyurea binding domain. The collagen binding domain is preferably an epidermal collagen binding domain. The keratin binding domain is preferably a keratin 1, 2, 3, 4, 5, 6, 7, or 8 binding domain. The cellulose binding domain is part of most cellulase enzymes and can be obtained therefrom. CBDs are also obtainable from xylanase and other hemicellulase degrading enzymes. Preferably, the cellulose binding domain is obtainable from a fungal enzyme origin such as Humicola, Trichoderma, Thermonospora, Phanerocfyaete, and Aspergillus, or from a bacterial origin such as Bacillus, Clostridium, Streptomyces, Cellulomonas and Pseudomonas species. Melamine binding domains are peptide/polypeptide binding domains that bind to melamine (a repeating unit of C₃N₆H₆ [1,3,5 triazine 2,4,6 triamine]) or a melamine-like polymer. Melamine polymers are commonly used as encapsulation materials.
It is most preferred that the heterologous tag is LacZ.

In connection with the present invention, the term "antimicrobial activity of dermcidin" denotes the antimicrobial activity of the C-terminal fragments of dermcidin (e.g., amino acids of SEQ ID No. 2 and SEQ ID No. 3) after proteolytic cleavage of the full-length dermcidin (SEQ ID No. 1) as described herein and any fragments thereof as long as they retain the antimicrobial activity of dermcidin (Figure 2, DCD1L, 110aa). It is preferred that the "peptide having the antimicrobial activity of dermcidin" of the 110 amino acids (aa) of dermcidin as shown in Figure 2 has the amino acid motif "SSLLEK" (amino acids 63 to 68 of dermcidin as shown in Figure 2) at its N-terminus or the amino acid motif "RSSLLEK" (amino acids 62 to 68 of dermcidin as shown in Figure 2) within the sequence.

There are several reasons that the nucleic acid molecule described herein encodes a fusion protein. The main reason being that the fusion protein is not antimicrobially active. It is not possible or at least highly disadvantageous to express an unfused nucleic acid molecule encoding a peptide having the antimicrobial activity of dermcidin in a bacterial host, since expression of such a nucleic acid molecule encoding a antimicrobial peptide or protein would be autotoxic to a host, like e.g., yeast, *E. coli* or *B. subtillis.* Only a peptide arising from the fusion protein described herein has the antimicrobial activity of dermcidin after proteolytic cleavage as described herein. Another reason is that most microorganisms, including *E*. *coli,* express small proteins and peptides only in inadequate amounts. Thus, expression as a fusion protein including e.g. a tag is expected to provide for increased expression.

The methods used until now in the art for expression of dermcidin and derivative peptides thereof as fusion proteins which where then cleaved off from their fusion partner display certain disadvantages. Cleavage was done by site-specific proteases like factor Xa [7] or by chemical cleavage with BrCN [8]. Although to the knowledge of the inventor not yet done, one could also design an expression construct harbouring a TEV-protease cleavage site for the activation of dermcidin derived peptides. These methods, currently used in the state of the art of fusion proteins, are disadvantageous since site specific proteases are expensive and cleavage with chemical reagents like BrCN creates by-products which have to be removed in further labour-intensive purification steps.

In contrast to the dermcidin fusion proteins known in the state of the art, the fusion protein described herein contains an arginine which is located immediately N-terminally of the peptide as defined in (b) above. This fusion protein, which is antimicrobially inactive, can be cut with an arginine specific protease at the position of the arginine located immediately N-terminally of the peptide of (b). Importantly, these finding is in contrast to the teaching of EP 1397384 B1 wherein it was not possible to proteolytically cleave dermcidin with a arginine-specific protease. By cutting off the peptide (tag) of (a) above from the fusion protein of the invention at this arginine position, the peptide of (b) above is capable to exert its antimicrobial activity.

Also described herein is a vector comprising the nucleic acid molecule of the invention and a host (cell) comprising said vector. Furthermore described herein is a method of producing the fusion protein encoded by the nucleic acid molecule described herein comprising culturing the host cell described herein and isolating the produced fusion protein.

Suitable methods for fusion protein isolation are described herein above in conjunction with the term "peptide isolation". Furthermore, it is preferred that the fusion protein described herein is obtained from the host cell using purification methods making use of the tag as defined herein. Means and methods for the isolation of a fusion protein using affinity protein-tags are described in Lichty et al. 2005 (Protein Expression and Purification Vol 41, Issue 1. p.98-105, "Comparision of affinity tags for protein purification"). Adding a proteinaceous tag to the peptide gives the fusion protein a binding affinity it would not otherwise have. Usually the recombinant protein is the only protein in the mixture with this affinity, which aids in separation. In case the tag is the Histidine-tag (His-tag), it has affinity towards nickel or cobalt ions. Thus by immobilizing nickel or cobalt ions on a resin, an affinity support that specifically binds to histidine-tagged peptides can be created. Since the fusion protein is the only component with a His-tag, all other proteins will pass through the column, and leave the His-tagged peptide bound to the resin. The fusion protein is released from the column in a process called elution, which in this preferred case involves adding imidazole, to compete with the His-tags for nickel binding, as it has a ring structure similar to histidine. The fusion protein of interest is now the major protein component in the eluted mixture. Another way to tag peptides is to engineer an antigen peptide tag onto the peptide, and then purify the fusion protein on a column or by incubating with a loose resin that is coated with an immobilized antibody. This particular procedure is known as immunoprecipitation. Immunoprecipitation is quite capable of generating an extremely specific interaction which usually results in binding only binding the desired peptide. The purified tagged peptide can then easily be separated from the other peptides in solution and later eluted back into clean solution. When the tags are not needed anymore, they can be cleaved off by a protease. This often involves engineering a protease cleavage site between the tag and the peptide. It is preferred that the protease is a arginine-specific protease and that the cleavage site is the arginine present between the peptide as defined in (b) and peptide as defined in (a) above.

The term "an arginine residue located immediately N-terminally of the peptide of (b)" as used herein, refers to an arginine-residue which is covalently linked via a peptide bond both to the peptide having the antimicrobial activity of (b) above and to the heterologous peptide of (a) above.

Also described herein is that the nucleic acid molecule described herein is a nucleic acid molecule wherein the peptide of (b) consists of amino acids 63 to 109 of dermcidin (SEQ ID NO: 2), of amino acids 63 to 110 of dermcidin (SEQ ID NO: 3), of amino acids 63 to 87 of dermcidin (SEQ ID NO: 4), of amino acids 63 to 85 of dermcidin (SEQ ID NO: 5), of amino acids 20 to 109 of dermcidin (SEQ ID NO: 6), or of amino acids 20 to 110 of dermcidin (SEQ ID NO: 7).

Full-length dermcidin is a polypeptide of 110aa amino acid (SEQ ID NO: 1, Fig 2). This dermcidin is proteolitically processed to C-terminal fragments of dermcidin having antimicrobial activity. Such peptides may consist of aa 63-110 (DCDL1, SEQ ID NO: 2), aa 63-109 (DCD1, SEQ ID NO: 3), aa 63-87 (SEQ ID NO: 4), or aa 63-85 (SEQ ID NO: 5) of dermcidin. These peptides have antimicrobial activity against several microorganisms like *E. coli,* methicillin-resistant *Staphylococcus aureus, Staphylococcus epidermidis* and *Candida albicans* [6]. SEQ.ID NOs: 2 to 5 do not contain the amino acid arginine. SEQ ID NO: 6 (aa 20 to 109 of dermcidin Figure 2) and SEQ ID NO: 7 (aa 20 to 110 of dermcidin in Figure 2) have a arginine in the position corresponding to aa positions 53, 59 and 62 of SEQ ID NO:1 (DCDL1 in Figure 2).

Also described herein is a fusion protein encoded by the nucleic acid molecule described herein or produced by the method described herein.

Also described herein is a method of producing an antimicrobial peptide comprising
(a) subjecting the fusion protein described herein to proteolytic cleavage with an arginine-specific protease and/or to proteolytic cleavage with a carboxypeptidase which cleaves off the C-terminal leucine in amino acid position 110 of dermcidin; and
(b) isolating the peptide having the antimicrobial activity of dermcidin from the cleavage product.

The term "arginine-specific protease" in accordance with the invention means a protease (EC 3.4.) which catalyzes the specific cleavage of a peptide at an arginine within the amino acid sequence. Arginine-specific proteases may be but are not limited to Clostripain, Gingipain and ArgC. Throughout this specification it is preferred that the arginine-specific protease is Clostripain or Gingipain, and even more preferred Clostripain.

Clostripain (EC 3.4.22.8) is an highly specific enzyme hydrolysing the carboxypeptide bond of arginine. It has been surprisingly found by the inventors that Clostripain selectively recognizes a sequence of the full-length dermcidin (SEQ ID No. 1) and any fragments thereof having an arginine at the amino acid position corresponding to amino acid position 62 of the full-length protein. Interestingly, the sequence required for specific cleavage by clostripain corresponds to the N-terminus of DCD1 or DCD1L (N-terminus of SEQ ID Nos. 2 or 3, respectively) peptide thus enabling to design a fusion protein as described herein without the need for the introduction of additional amino acids to provide for a specific cleavage site for a protease.

As described herein above the fusion protein described herein has no antimicrobial activity and only exerts the antimicrobial activity of the peptide as defined in (b) above after proteolytic cleavage with a arginine-specific protease and/or a carboxypeptidase which cleaves off the C-terminal leucine in amino acid position 110 of dermcidin.

A further preferred embodiment of the method of the invention further comprises purifying the peptide preferably the peptide isolated from the resulting cleavage product to homogeneity.

The term "homogeneity" refers to the degree of purity of the isolated peptide, preferably the antimicrobial peptide. It is preferred that the homogeneity is sufficient to use the antimicrobial peptide in a pharmaceutical composition or a cosmetic composition. With increasing preference the degree of purity of the antimicrobial peptide is at least 50%, at least 75%, at least 90%, at least 95%, at least 98%, at least 99%, at least 99,9% or even 100%.

Methods for purifying a peptide to homogeneity are known in the state of the art and include protein precipitation, ultracentrifugation or chromatographic (e.g. size exclusion chromatography or RP-HPLC) methods. Such methods can easily separate desired from any minor unwanted contaminants after peptide isolation. Further steps for the concentration of the peptide may be also included.

In a particularly preferred embodiment the method of the invention further comprises the step of formulating the peptide preferably with antimicobially activity with a pharmaceutically acceptable carrier, diluent or excipient. As mentioned, if the peptide does not have antimicrobial activity, it can be converted such an embodiment by enzymatic cleavage.

Pharmaceutically acceptable carriers, diluents or excipients are known to the skilled person and are for example described in Ansel et al., "Pharmaceutical Dosage Forms and Drug Delivery Systems", 7th edition, Lippincott Williams & Wilkins Publishers, 1999. Further, means and methods of pharmaceutically acceptable carriers, diluents or excipient are described herein in conjunction with a pharmaceutical composition.

In another particularly preferred embodiment of the invention method further comprises the step of admixing the peptide preferably with antimicobially activity to a skin benefit agent or dermatological benefit agent.

"Skin benefit agents and dermatological benefit agents" in accordance with the invention include:
(a) silicone oils and modifications thereof such as linear and cyclic polydimethylsiloxanes; amino, alkyl, alkylaryl, and aryl silicone oils;
(b) fats and oils including natural fats and oils such as jojoba, soybean, sunflower, rice bran, avocado, almond, olive, sesame, persic, castor, coconut, mink oils; cacao fat; beef tallow, lard; hardened oils obtained by hydrogenating the aforementioned oils; and synthetic mono, di- and triglycerides such as myristic acid glyceride and 2-ethylhexanoic acid glyceride;
(c) waxes such as carnauba, spermaceti, beeswax, lanolin, and derivatives thereof;
(d) hydrophobic and hydrophilic plant extracts;
(e) hydrocarbons such as liquid paraffins, vaseline, microcrystalline wax, ceresin, squalene, pristan and mineral oil;
(f) higher fatty acids such as lauric, myristic, palmitic, stearic, behenic, oleic, linoleic, linolenic, lanolic, isostearic', arachidonic and poly unsaturated fatty acids (PUFA);
(g) higher alcohols such as lauryl, cetyl, stearyl, oleyl, behenyl, cholesterol and 2-hexydecanol alcohol;
(h) esters such as cetyl octanoate, myristyl lactate, cetyl lactate, isopropyl myristate, myristyl myristate, isopropyl palmitate, isopropyl adipate, butyl stearate, decyl oleate, cholesterol isostearate, glycerol monostearate, glycerol distearate, glycerol tristearate, alkyl lactate, alkyl citrate and alkyl tartrate;
(i) essential oils and extracts thereof such as mentha, jasmine, camphor, white cedar, bitter orange peel, ryu, turpentine, cinnamon, bergamot, citrus unshiu, calamus, pine, lavender, bay, clove, hiba, eucalyptus, lemon, starflower, thyme, peppermint, rose, sage, sesame, ginger, basil, juniper, lemon grass, rosemary, rosewood, avocado, grape, grapeseed, myrrh, cucumber, watercress, calendula, elder flower, geranium, linden blossom, amaranth, seaweed, ginko, ginseng, carrot, guarana, tea tree, jojoba, comfrey, oatmeal, cocoa, neroli, vanilla, green tea, penny royal, aloe vera, menthol, cineole, eugenol, citral, citronelle, borneol, linalool, geraniol, evening primrose, camphor, thymol, spirantol, penene, limonene and terpenoid oils;
(j) lipids such as cholesterol, ceramides, sucrose esters and pseudo- ceramides as described in European Patent Specification No. 556,957;
(k) vitamins, minerals, and skin nutrients such as milk, vitamins A, E, and K; vitamin alkyl esters, including vitamin C alkyl esters; magnesium, calcium, copper, zinc and other metallic components;
(l) sunscreens such as octyl methoxyl cinnamate (Parsol MCX) and butyl methoxy benzoylmethane (Parsol 1789) ;
(m) phospholipids;
(n) antimicrobial agents such as the heavy metal salts of pyridinethione, climbazole, piroctone olamine, selenium sulphide and ketoconazole;
(o) antiaging compounds such as alpha hydroxy acids, beta hydroxy acids;
(p) pigment particles, such as solid dyes or colorants
(q) opacifying agents including higher fatty alcohols (e.g. cetyl, stearyl, arachidyl and behenyl), solid esters (e.g. cetyl palmitate, glyceryl laurate, stearamide MEA-stearate), high molecular weight fatty amides and alkanolamides and various fatty acid derivatives such as propylene glycol and polyethylene glycol esters. Inorganic materials include magnesium aluminium silicate, zinc oxide, and titanium dioxide.
(r) Pearlescing agents such as C16-C22 fatty acids (e. g. stearic acid, myristic acid, oleic acid and behenic acid), esters of C16-C22 fatty acid with alcohols and esters of C16- C22 fatty acid incorporating such elements as alkylene glycol units. Suitable alkylene glycol units may include ethylene glycol and propylene glycol. However, higher alkylene chain length glycols may also be employed. Suitable higher alkylene chain length glycols include polyethylene glycol and polypropylene glycol. Further suitable pearlescing agents include inorganic materials such as nacreous pigments based on the natural mineral mica. An example is titanium dioxide coated mica. Particles of this material may vary in size from 2 to 150 microns in diameter. In general, smaller particles give rise to a pearly appearance, whereas particles having a larger average diameter will result in a glittery composition.
(s) antioxidants;
(t) fragrances and/or perfumes; and
(u) mixtures of any of the foregoing components.

The amount of skin benefit agent is preferably from 0.001 to 15 wt%, such as from 0.01 wt% to 10 wt%.

The skin and dermatological benefit agent may be encapsulated in a melamine capsule. The production of melamine capsules is well known in the art, see for instance WO01/51197, WO01/49817, US6,248,703. They contain and are characterised by the repeating unit of C₃N₆H₆ [1,3,5 triazine 2,4,6 triamine]. These melamine polymers are advantageously used in the manufacture of micro-capsules, preferably having a particle size of between 0.1 and 100 µm, more preferably of between 10 and 50 µm. Such micro-capsules are well known and have been described in US-A-2003078043, JP-A-10139817, WO03/035245, US6,080,418. These melamine-polymer containing micro-capsules contain the skin and dermatological benefit agents. Many processes for microencapsulation are known. These include methods for capsule formation such as described in US2, 730,456, US2, 800,457 and US2,800,458. Other useful methods for microcapsule manufacture are described in: US4, 001,140, US4, 081,376 and US4,089,802 describing a reaction between urea and formaldehyde; US4,100,103 describing reaction between melamine and formaldehyde; GB2, 062,570 describing a process for producing microcapsules having walls produced by polymerisation.of melamine and formaldehyde in the presence of a styrenesulfonic acid. Micro-encapsulation is also taught in US-A-2 730 457 and US-A-4 197 346.

In a particularly preferred embodiment the method of the invention further comprises packaging the product obtained in unit dosage form.

A "unit dosage form" in accordance with the invention refers to a composition intended for a single administration to treat a subject suffering from a disease or medical condition. Examples of unit dosage forms are individual tablets, individual gelatin capsules, bulk powders, and liquid solutions, emulsions or suspensions. It is preferred that the unit dosage form is a emulsion or suspension which is applied to the skin of a subject (transdermal application). Treatment of the diseases or conditions described herein may require periodic administration of unit dosage forms, for example: one unit dosage form two or more times a day, one with each meal, one every four hours or other interval, or only one per day. The concentration of the peptide produced by the method of the invention is in the range 1 -50 µg/ml, preferably 0.1 - 100 µg/ml, or even more preferred 0.01 -1000 µg/ml.

Also described herein is a composition comprising
(a) the fusion protein described herein or the peptide produced by the method of the invention, and
(b) optionally a arginine-specific protease and/or a carboxypeptidase which cleaves off the C-terminal leucine in amino acid position 110 of dermcidin.

In this regard, it has to be understood that the catalytic activation of the the arginine-specific protease and/or carboxypeptidase of (b) confers the antimicrobial activity of the fusion-protein or peptide of (a) by proteolytic cleavage. In other words, the combination of a arginine-specific protease and/or a carboxypeptidase with the fusion protein or peptide described herein makes the composition described herein an antimicrobial composition.

The antimicrobial composition ensues that - upon contact of the fusion protein or the above peptide with a arginine-specific protease and/or a carboxypeptidase which cleaves off the C-terminal leucine in amino acid position 110 of dermcidin, or likewise upon proteolytical processing of the fusion protein or peptide on the skin of a subject by proteases present on the skin (see examples 3 und 4) - is suitable to kill microorganisms (herein interchangeably used with microbes) (microbicidal composition) or to inhibit the growth of microorganisms (microbistatic composition), including bacteria and fungi. The composition may thus be an antibiotic or an anti-fungal composition. It is preferred that these microorganisms are pathogenic microorganisms or microorganism which have unwanted effect on the appearance or condition of the skin.

Also described herein is that in the composition described herein the fusion protein or the peptide of a) is to be contacted with the arginine-specific protease and/or the carboxypeptidase of b) on the skin of a subject.

Also described herein is a method comprising contacting the fusion protein or the peptide of a) above with the arginine-specific protease and/or the carboxypeptidase of b) on the skin of a subject.

The term "subject" in accordance with the invention refers to an animal which can be preferably a vertebrate, more preferably a mammal and most preferably a domestic animal or a pet animal such as horse, cattle, pig, sheep, goat, dog or cat, and most preferably a human. Also, the subject may be a fish or bird such as a chicken.

The term "skin" in accordance with the invention is the outer covering of the body of a subject. The skin may include skin appendages like hairs, feather, glands and nails. In particular open areas of the skin, like wounds, healing wounds and skin damages are also envisaged.

This method allows for the activation of the antimicrobial activity on the skin of a subject. This may be in particular advantageous to control the antimicrobial activity in place and time. In general, the fusion protein is more stable over time compared to the antimicrobial peptide generated by the arginine-specific proteolytic cleavage. Thus, it might be advantageous to just cleave the fusion protein directly before the treatment takes place. Next to harmful bacteria and fungi, there are also bacteria and fungi on the skin which are harmless or even beneficial. Therefore, it is highly advantageous to confine the treatment to the place of infection.

In methods or the composition described herein the arginine-specific protease may be Clostripain or Gingipain R.

Also described herein is the composition of the invention for use in preventing or treating a microbial skin infection.

Also described herein is a method of treating a patient having a microbial skin infection by administering to said patient an pharmaceutically effective amount of the composition described herein.

In accordance with the invention microbial skin infection are caused by the presence and/or growth of microorganisms (i.e. bacteria and fungi) that damage host tissue. The term "skin infections" includes infections of the skin, hair follicle, sweat glands, sebaceous glands and nail bed. Microbial skin infections are very common, and they can range from merely annoying to deadly. Microbial skin infections can be caused by bacteria such as *Staphylococcus aureus, Streptococcus,* Commensals (e.g., *Corynebacterium, micrococci, P. acnes*), myobacteria, or *Pseudomonas.* Most microbial skin infections are caused by two bacteria, *Staphylococcus aureus* and *Streptococcus pyogenes.* Microbial skin infections caused by bacteria include but are not limited to Impetigo, Ecthyma, Folliculitis (e.g. Abscess, Furuncles, and Carbuncles Staphyloccocal Scalded Skin Syndrome), Toxic Shock Syndrome, Secondary infections (e.g. atopic dermatitis, cuts and Scrapes, wound care), MRSA (methicillin resistant staph aureus), Cellulitis, Impetigo, Ecthyma, Erysipelas, Scarlet Fever, Necrotizing Fasciitis, Streptococcal Peri-anal Disease, Streptococcal Toxic Shock Syndrome, Erythrasma, Pitted keratolysis, Trichomycosis axillaris, Leprosy, Skin Tuberculosis, Atypical Mycobacteria, Folliculitis, Pyoderma, ear infections, Green Nail Syndrome, Spirochetes, Lyme Disease, Rickettsial Disease, Spotted Fever, and acute and chronic Meningococcemia.

Moreover, microbial skin infections may be caused by fungi. Microbial skin infections caused by fungi are also known as dermatomycosis and include but are not limited to Tinea superficialis, Tinea pedis, Tinea unguis, Tinea pofunda, Tinea barbae, infections of the mucosa membrane of nose, mouth, fauces, digestive tract and genitals. Fungi causing microbial skin infections include but are not limited to the genus *Candida* (i.e. *Candida albicans*), *Cryptococcus neoformans,* the genus *Aspergillus, Microsporum, Trichophyton,* and *Epidermophyton* fungi.

Also described herein is the composition described herein for use in preventing or treating cancer or tuberculosis. The role of dermicidin in cancer and tuberculosis is described in [7].

The composition described herein may be a pharmaceutical composition.

The "pharmaceutical composition" described herein is used to treat or prevent a disease and in particular microbial skin infections as described herein above. The pharmaceutical composition can be formulated in conventional manner according to methods found in the art, using one or more physiological carriers or excipient, see, for example Ansel et al., "Pharmaceutical Dosage Forms and Drug Delivery Systems", 7th edition, Lippincott Williams & Wilkins Publishers, 1999. The pharmaceutical composition may, accordingly, be administered orally, parenterally, such as subcutaneously, intravenously, intramuscularly, intraperitoneally, intrathecally, transdermally, transmucosally, subdurally, locally or typically via iontopheresis, sublingually, by inhalation spray, aerosol or rectally and the like in dosage unit formulations optionally comprising conventional pharmaceutically acceptable excipients. Systemic applications of the pharmaceutical composition described herein are in particular preferred in a subject having a systemic infections and local application of the pharmaceutical composition described herein are in particular preferred in a subject having a locally restricted infection.

The pharmaceutical composition described herein is preferably formulated for transdermal administration. Transdermal compositions are typically formulated as a topical gel, powder, ointment or cream containing the active ingredient(s), generally in an amount ranging from about 0.01 to about 20% by weight, preferably from about 0.1 to about 20% by weight, preferably from about 0.1 to about 10% by weight, and more preferably from about 0.5 to about 15% by weight. The transdermal composition may combined with a band aid, dressing or tape for the application to the skin. The concentration of the peptide produced by the method of the invention in a composition to be immobilised in a matrix of a dressing the like should be in the range of 1 -50 µg/ml, preferably 0.1 - 100 µg/ml, or even more preferred 0.01 - 1000 µg/ml.

When formulated as an ointment, the active ingredients will typically be combined with either a paraffinic or a water-miscible ointment base. Alternatively, the active ingredients may be formulated in a cream with, for example an oil-in-water cream base. Such transdermal formulations are well-known in the art and generally include additional ingredients to enhance the dermal penetration of stability of the active ingredients or the formulation. All such known transdermal formulations and ingredients are included. The compounds or compositions described herein can also be administered by a transdermal device. Accordingly, transdermal administration can be accomplished using a patch either of the reservoir or porous membrane type, or of a solid matrix variety.

The pharmaceutical composition described herein can be formulated as neutral or salt forms. Pharmaceutically acceptable salts include those formed with anions such as those derived from hydrochloric, phosphoric, acetic, oxalic, tartaric acids, etc., and those formed with cations such as those derived from sodium, potassium, ammonium, calcium, ferric hydroxides, isopropylamine, triethylamine, 2-ethylamino ethanol, histidine, procaine, etc.

The pharmaceutical composition described herein can also, if desired, be presented in a pack, or dispenser device which can contain one or more unit dosage forms containing the agent. The pack can for example comprise metal or plastic foil, such as blister pack. The pack or dispenser device can be accompanied with instruction for administration.

The pharmaceutical composition described herein can be administered as sole active agent or can be administered in combination with other agents.

The pharmaceutical composition is formulated generally by mixing it at the desired degree of purity, in a unit dosage form (solution, suspension, or emulsion), with a pharmaceutically acceptable carrier, i.e., one that is non-toxic to recipients at the dosages and concentrations employed and is compatible with other ingredients of the formulation.

Generally, the formulations are prepared by contacting the components of the pharmaceutical composition uniformly and intimately with liquid carriers or finely divided solid carriers or both. Then, if necessary, the product is shaped into the desired formulation. Examples of such carrier vehicles include water, saline, Ringer's solution, and dextrose solution. Non aqueous vehicles such as fixed oils and ethyl oleate are also useful herein, as well as liposomes. The carrier suitably contains minor amounts of additives such as substances that enhance isotonicity and chemical stability. Such materials are non-toxic to recipients at the dosages and concentrations employed, and include buffers such as phosphate, citrate, succinate, acetic acid, and other organic acids or their salts; antioxidants such as ascorbic acid; low molecular weight (less than about ten residues) polypeptides, e.g., polyarginine or tripeptides; polypeptides, such as serum albumin, gelatin, or immunoglobulins; hydrophilic polymers such as polyvinylpyrrolidone; amino acids, such as glycine, glutamic acid, aspartic acid, or arginine; monosaccharides, disaccharides, and other carbohydrates including cellulose or its derivatives, glucose, mannose, or dextrins; chelating agents such as EDTA; sugar alcohols such as mannitol or sorbitol; counterions such as sodium; and/or nonionic surfactants such as polysorbates, poloxamers, or PEG.

The components of the pharmaceutical composition to be used for therapeutic administration must be sterile. Sterility is readily accomplished by filtration through sterile filtration membranes (e.g., 0.2 micron membranes). Therapeutic components of the pharmaceutical composition generally are placed into a container having a sterile access port, for example, an intravenous solution bag or vial having a stopper pierceable by a hypodermic injection needle. The components of the pharmaceutical composition ordinarily will be stored in unit or multidose containers, for example, sealed ampoules or vials, as an aqueous solution or as a lyophilized formulation for reconstitution. As an example of a lyophilized formulation, 10-ml vials are filled with 5 ml of sterile-filtered 1% (w/v) aqueous solution, and the resulting mixture is lyophilized. The solution is prepared by reconstituting the lyophilized compound(s) using bacteriostatic Water-for-Injection.

The composition described herein may be a cosmetic composition.

The "cosmetic composition" as described herein is used to enhance the appearance of the skin of a subject, cleaning of the skin, to achieve or reconstitute the skin balance and the like. Cosmetic compositions comprise creams, lotions, powders, perfumes, lipsticks, fingernail and toe nail polish, eye and facial makeup, hair colors, hair sprays and gels, tooth pastes, deodorants, baby products, bath oils, bubble baths, bath salts, and butters. The concentration of the peptide isolated by the methods of the invention should be in the range of 1 -50 µg/ml, preferably 0.1 - 100 µg/ml, or even more preferred 0.01 - 1000 µg/ml. Suitable additional ingredients for a cosmetic composition comprise the ingredients listed in the *International Nomenclature of Cosmetic Ingredients* (http://ec.europa.eu/enterprise/cosmetics/inci/inci_2006.pdf) or the US *Chemistry, Toiletry and Fragrance Association Nomenclature.*

The Figures show:
Figure 1 shows the SDS PAGE (A) and western blot (B) analysis of DCD1 and DCD1 L.
Figure 2 shows the sequence of dermcidin. Depicted in italic letters is the signal peptide for the secretion in eukaryotic cells. Bold indicates the pro peptide which is removed for activation of DCD1L peptides. Underlined the mature DCD1 L and DCD1 peptide is shown.
Figure 3A shows the protein sequence of the LacZ-dermcidin fusion protein. Depicted in italic letters is the fusion protein LacZ. The R-residue highlighted bold is the relevant clostripain processing site necessary for the activation of the peptide. Underlined the mature DCD1 L and DCD1 peptide is shown.
Figure 3B depicts the vector map of the pET based expression construct for the production of LacZ-dermcidin fusion proteins.
Figure 4 shows an antimicrobial assay-2 against *S*. *aureus* with DCD1 in different concentrations. DCD1 was dissolved in 0.06 % FA (2-5) or 40% ACN/ 0.06% FA (7-10). 1. 0.06% FA; 2. 5µg DCD1; 3. 10 µg DCD1; 4. 30 µg DCD1; 5. 60 µg DCD1; 6. 40% ACN/ 0.06% FA; 7. 5µg DCD1; 8. 10 µg DCD1; 9. 30 µg DCD1; 10. 60 µg DCD1.
Figure 5 shows the SDS PAGE of the DCD1L processing. Line 1-3: DCD1L dissolved in 300 mM NaCl, 10 mM NaH₂PO₄, pH 4.0 and incubated on skin for 0, 2 and 4 hours; Line 4-6: DCD1L dissolved in 300 mM NaCl, 10 mM NaH₂PO₄, pH 6.5 and incubated on skin for 0, 2 and 4 hours; Line 1-3: DCD1L dissolved in 300 mM NaCl, 10 mM NaH₂PO₄, pH 9.0 and incubated on skin for 0, 2 and 4 hours. As controls are the recombinantly produced DCD1 and DCD1L peptides.
Figure 6 shows the time dependent conversion of DCD1L to DCD1 using HPLC-MS. (A) 2h, (B) 4h, (C) 19h. A total ion count chromatogram (MIC) is shown in black and two single ion chromatograms for m/z 785 and m/z 805 corresponding to DCD1 and DCD1L respectively are shown.
Figure 7 shows the pH and time dependency of DCD1L conversion to DCD1
Figure 8 shows an antimicrobial assay-2 against *S*. *aureus* with DCD1 recombinantly produced or by *in vivo* processing of DCD1L. DCD1 was dissolved in 40% ACN/ 0.06% FA. 1. 24 µg recombinantly produced DCD1 2. 24 µg *in vivo* produced DCD1; 3. 10 µl 40% ACN/ 0.06% FA;
Figure 9 shows the comparison of the DCD1L processing of men and women.
Figure 10 shows the SDS PAGE (4 - 12% Invitrogen NuPAGE) analysis of 10µl of supernatants from a *Pichia pastoris* fermentation. Timepoints are 69 hours (1), 51.5 h (2), 50 h (3), 47.5 h (4), 45 h (5), 42 h (6) and 23 h (7). Molecular weight markers (M) in side panel in kDa. Gel band identified as ProDCD1L is indicated by arrow.
Figure 11 shows the Mascot search results generated by PANATecs GmbH (Tübingen).

The Examples illustrate the invention:

### Example 1: Experimental materials and methods

### Recombinant expression in E. coli as host

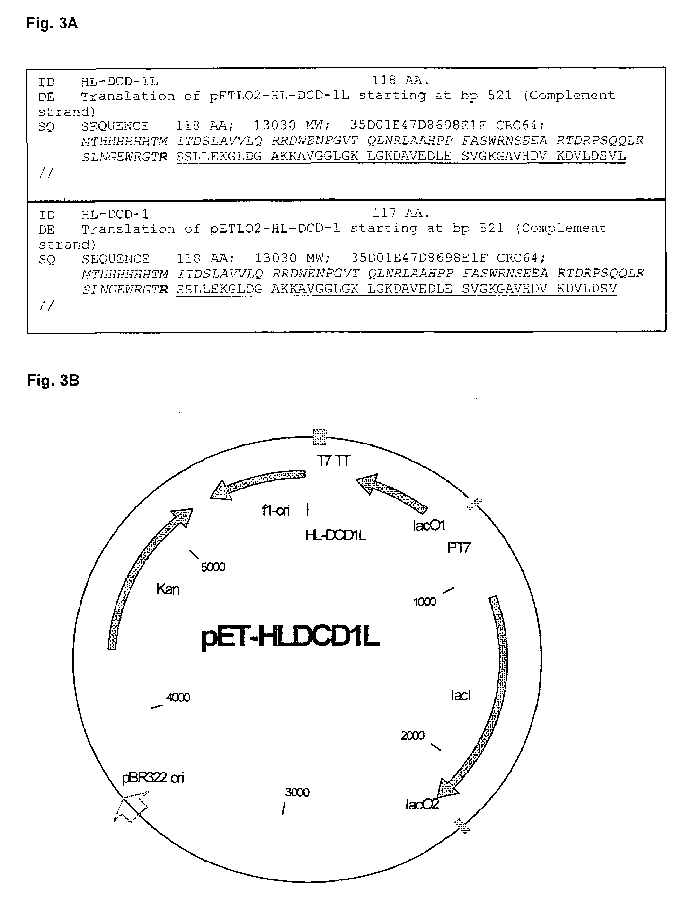

For heterologous expression of antimicrobial peptides and fusion proteins (Fig 3, A and B) *E. coli* BL21 (Novagen) was used. A single bacterial colony was inoculated in rich liquid LB medium pre-culture containing the appropriate antibiotic and 2 % glucose. After 16h growth at 37° C the pre-culture was used to inoculate fresh LB medium containing the appropriate antibiotic and 2 % glucose at an optical density (OD580) of 0.05. Cultures where then grown at 28° C until the optical density reached a value of 1.0 (OD580). Cells were then induced with isopropyl-β-D-thiogalactopyranosid (IPTG) in a final concentration of 200 µM. 4h after induction cells were harvested by centrifugation.

### Recombinant expression in yeast as host

For heterologous expression of antimicrobial peptides *Pichia pastoris* was used. A construct comprising a suitable secretion signal and the coding sequence of DCD1L was introduced into the host cell under the control of the AOX promoter. For the expression a single yeast colony was inoculated in rich liquid YPD medium as pre-culture. After 16h growth at 30° C the pre-culture was used to inoculate a bioreactor (NFL19, Bioengineering AG) containing 6 L mineral salt medium [11] at an optical density (OD580) of 1. Cultures where then grown at 28° C until dissolved oxygen level indicated the end of the batch phase. Cells were then induced with a continuous feed of 50% Glycerol, 20% Methanol in water. Cells were cultivated until they reached a suitable cell density and the supernatant was then harvested. DCD1L was purified from the supernatant by HPLC.

### Purification of Fusion proteins

Cells from a 300 ml LB (Kan, Glc) culture were resuspended in 4 ml lysis buffer (50 mM NaH₂PO₄, 300 mM NaCl, 10 mM imidazol, 6M Urea, 1 mg /ml lysozyme pH 8.0) and incubated for 30 min on ice. The cells were lysed by sonification (3 x 90s, 100W, output 4, 50% duty) on ice. The lysate was clarified by centrifugation for 30 min at 16000 x g and subsequent filtration with a 0.22 µm filter.

The fusion proteins were purified out of the cleared lysate by Immobilized Metal Affinity Chromatography (IMAC) on Aekta-Explorer FPLC-system. NTA-Superflow (GE Healthcare) was used as chromatography matrix. The flowrate was 0.2 ml/min and 4 ml of sample were injected. A 4 step gradient using 3 different buffer systems was used. Buffer A1 (50 mM NaH₂PO₄, 300 mM NaCl, 10 mM imidazol, 6M urea, pH 8.0), buffer A2 (50 mM NaH₂PO₄, 30 0mM NaCl, 10 mM imidazol, pH 8.0) and buffer B (50 mM NaH₂PO₄, 300 mM NaCl, 500 mM imidazol, pH 8.0). The gradient was comprised of 5 column volumes (CV) 100% buffer A1, 8 CV 90% buffer A1/10% buffer A2, 8 CV 90% buffer A2/10% buffer B and 8 CV 100% buffer B. Product was eluted in the last gradient step (100% buffer B).

After purification the eluate was dialyzed against storage buffer (50 mM Na₂HPO₄, 30 mM NaCl, pH 7.5).

### Detection and Purification of DCD1L

A Jasco 980 HPLC system was used to monitor the processing. Samples were subjected to RP-HPLC, on a Purospher RP-18e 125-4 column (Merck) with 5 µm particle size and 100 A pore size. Solvent A was H₂O / 0.1 % TFA; solvent B was Acetonitrile/0.1 % TFA. A linear gradient of 5 % B to 100 % B over 27 min with a flow rate of 1 ml/min was used.

For the purification a preparative Luna C18 (2) 250-10 column (Phenomenex) with a 5 µm particle size and a 100 A pore size was used. Solvent A was H₂O / 0.1 % TFA; solvent B was Acetonitrile/0.1 % TFA. A linear gradient of 5 % B to 100 % B over 27 min with a flow rate of 6.25 ml/min was used.

### Antimicrobial activity assay - 1

Antimicrobial assays were performed using the colony-forming units (CFU) assay as previously described [6]. The antibacterial activity of DCD-derived peptides was tested against the following bacterial strains: *Escherichia coli* and *Staphylococcus aureus.* Bacterial cultures were grown to mid-exponential growth phase and washed three times with 10 mM sodium phosphate buffer/10 mM NaCl (pH 7.0). Bacterial concentration was estimated photometrically at 600 nm. Absorbance of 1.0 corresponded to 8.56 x 10⁸/ml for *E*. *coli,* 1.97 x 10⁸/ml for *S*. *aureus.*

After dilution to a concentration of 10⁶ CFU/ml, 10 µl of the dilutions were incubated at 37°C for 2-4 hours with the respective peptide diluted in water in a total volume of 30 µl in 10 mM sodium phosphate buffer containing 10 mM NaCl (pH 7.0). After incubation, cells were diluted 1:100 in 10 mM sodium phosphate buffer containing 10 mM NaCl (pH 7.0) and 90 µl of the diluted bacterial suspension were plated in triplicates on blood agar. Bacterial colonies were counted after incubation for 18-24 hours at 37° C. The antimicrobial activity was calculated using [(cell survival after peptide incubation)/(cell survival in buffer without peptide) x 100]. The LC90 describes the lethal concentration of the current peptide in µg/ml or M which leads to 90% reduction of colony-forming units compared to the buffer control. The assay was carried out with 50 - 100 µg/ml DCD1L and DCD1. Surprisingly only the addition of DCD1 led to a reduction of diverse bacteria.

### Antimicrobial activity assay - 2

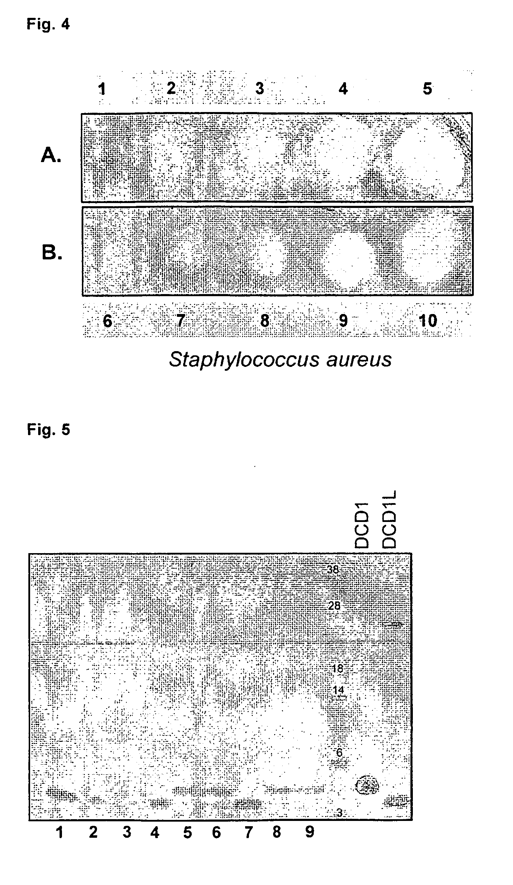

The antimicrobial activity of DCD1 and DCD1L peptides were tested against the following bacterial strains: *Escherichia coli, Staphylococcus aureus,* and *Micrococcus luteus,* isolated from skin. Bacterial cultures were grown to mid-exponential growth phase and bacterial concentration was estimated photometrically at 578 nm. Absorbance of 1.0 corresponded to 8.56 x 10⁸/ml for *E. coli,* 1.97 x 10⁸/ml for *S*. *aureus.* After dilution to a concentration of OD₅₇₈ₙₘ 0.01 (∼10⁶ CFU/ml), 100 µl of the cells were plated on LB agar. Afterwards different concentrations of the peptide dissolved in 40 % ACN/ 0.06 % FA were spotted on the plates in a total volume of 10 µl. As control 10 µl of 40 % ACN/ 0.06 % FA was used. The plates were incubated at 37°C over night. After incubation the zone of inhibition was determined and the antimicrobial activity was calculated in dependence on the peptide concentration. In accordance with the antimicrobial activity assay-1 only activity emanating from DCD1 was detectable (Fig 4).

### Recombinant expression in yeast as host

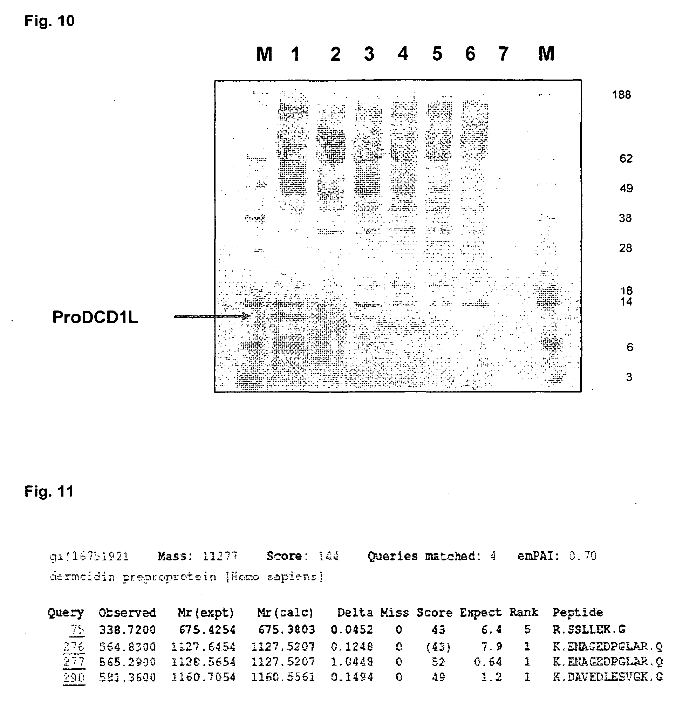

For heterologous expression of antimicrobial peptides *Pichia pastoris* was used. A construct comprising a suitable secretion signal and the coding sequence of proDCD1L was introduced into the host cell under the control of the AOX promoter. For the expression a single yeast colony was inoculated in rich liquid YPD medium as pre-culture. After 16h growth at 30° C the pre-culture was used to inoculate a bioreactor (NFL19, Bioengineering AG) containing 6 L mineral salt medium (per L: 2 g citric acid, 12.4 g (NH₄)₂HPO₄, 0.9 g KCI, 0.5 g MgSO₄ x 7H₂O, 40 g glycerol) at an optical density (OD580) of 1. Cultures where then grown at 28° C until dissolved oxygen level indicated the end of the batch phase. Cells were then induced with a continuous feed of 50% glycerol, 20% methanol in water. Cells were cultivated until they reached maximal cell density and the supernatant was harvested. proDCD1L was purified from the supernatant by ion exchange chromatography. For further analysis proDCD1L was analyzed by SDS PAGE (Fig. 10) and the corresponding gel band was further analyzed by PANATecs GmbH (Tübingen, Germany) using a nano LC-ESI-MS/MS method after tryptic digest of the gel band. Corresponding Mascot search results (PANATecs GmbH, Tübingen) are shown in Figure 11.

### Example 2: Processing of Fusion proteins with an Arg-Specific Protease

Prior to processing the fusion protein, lyophilized clostripain (Sigma) was activated by the incubation at 28° C in activation buffer (10 mM MOPS, 2.5 mM DTT, 1 mM CaCl₂, pH7.4) for 3 h. Final concentration of clostripain was 1 U/µl.

Purified fusion protein at a concentration of 1 mg / ml was incubated with 0.05 U/ml activated clostripain in processing buffer (50mM Na₂HPO₄, 30mM NaCl, 2.5 mM DTT, 10 µM CaCl₂, pH 7.5) for 16 h at 28° C. These parameters have to be evaluated for every protease and fusion protein used. In table 1 some examples of valid parameter combinations are listed.

**Table 1: Parameter combinations for processing of dermcidin fusionproteins**

| Fusion protein concentration [mg/ml] | Clostripain amount [U/µl] | Incubation time [h] |
|---|---|---|
| 1 | 0.05 | 16 |
| 20 | 0.5 | 0.5 |
| 20 | 0.25 | 1.5 |

### Example 3: Detection of processed DCD1 and DCD1L

After processing and purification the peptides were separated by SDS PAGE [9] (Fig 1A) and analyzed by western blotting with DCD-specific antibodies (Fig 1 B). Interestingly both peptides show different pattern using Bis-Tris PAA gels. DCD1 (47 aa) has a lower migration in the gel than DCD1L (48 aa) although representing the smaller peptide. This can be explained by conformational differences between DCD1 and DCD1L, which are essential for antimicrobial activity.

For further analysis DCD1 was transferred after SDS PAGE to a PVDF membrane by electroblotting [10]. Correct processing of DCD1 was determined by N-terminal Edman sequencing by Protagen AG (Dortmund).

For Edman sequencing the blot was fitted into the sample preparation cartridge. For determination of the amino acid sequence the protein sequencer Procise 492 (Applied Biosystems) was used. Reagents and protocols were applied as advised by the manufacture. The resulting chromatograms were analyzed using appropriate software (Applied Biosystems). Prior to each sample a standard sample and a blank were run.

The analysis determined two major sequences in the sample. One N-terminal sequence determined was SSLLEKGL which corresponds to the expected N-terminus (Fig 2, Fig 3A and 3B). The other was THHHHHHT which corresponds to the N-terminus of the fusion protein.

### Example 4: In vivo processing of DCD1

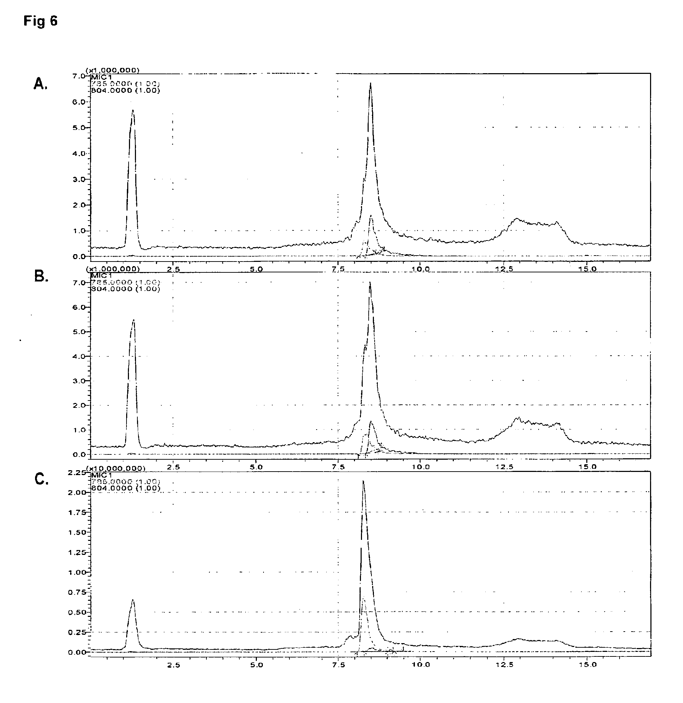

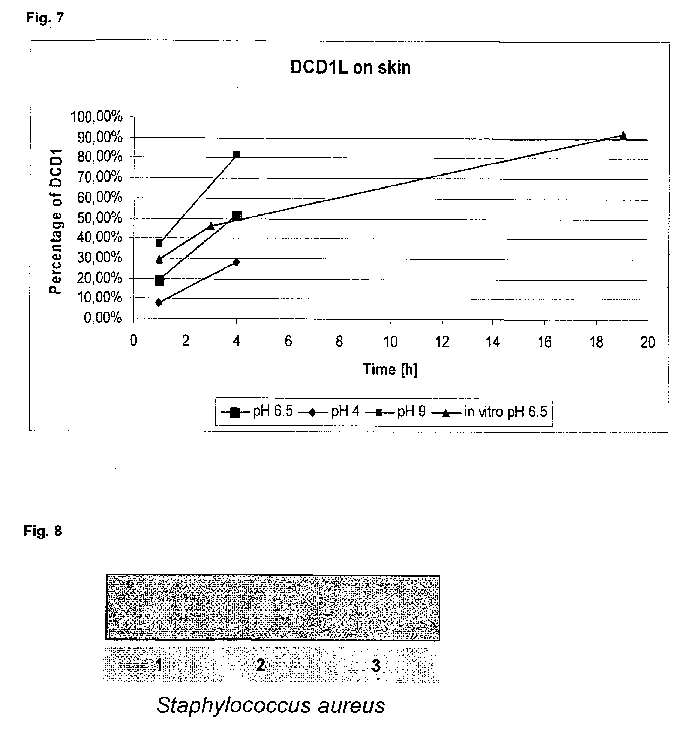

For testing *in vivo* processing, the DCD1L peptide was dissolved in buffers (300 mM NaCl, 10 mM NaH₂PO₄) varying in pH (4, 6.5, and 9) and incubated on forearm. Therefore a construction composed of cardboard containing five ceramic rings was created. This construction was fixed on forearm with tape and ceramic rings were filled with 100 µl (∼140 µg) DCD1L. Finally an elastic foil was used to surround the whole construction. The incubation on skin varied from one to four hours before removing the samples from skin. Afterwards the peptide samples were analyzed by SDS PAGE (Fig. 5) and HPLC-MS (Fig 6 A and B) and also further incubated at room temperature *in vitro* (Fig 6 C). A second approach was performed by incubating the buffer 300 mM NaCl, 10 mM NaH₂PO₄, pH 9 on forearm up to four hours. After removal from skin, the buffer was suplemented with DCD1L peptide and incubated *in vitro* over night. This kind of processing was also analyzed by SDS PAGE and HPLC-MS. The analysis of the *in vivolin vitro* DCD processing showed that DCD1L is always converted into DCD1 in a pH- and time-dependent manner (Fig 7). The processing of DCD1L to DCD1 was faster using buffer with pH 9 and was complete after incubation for 16 h (Fig 6C).

To determine the antimicrobial activity, the *in vivo* produced DCD1 was lyophilized and dissolved in 40% ACN/ 0.06 % FA. For testing its activity - 28 µg DCD1 was spotted in LB agar plates containing 100 µl *S*. *aureus* (OD₅₇₈ₙₘ 0.01) (see chapter Antimicrobial activity assay - 2). As control 40% ACN/ 0.06% FA was spotted. The determination of the inhibition zone demonstrated that DCD1, formerly DCD1L and processed on skin, is antimicrobial active. 40% ACN/ 0.06% FA shows no activity (Fig. 8).

### Example 5: In vivo processing of DCD1 - the difference between men and women

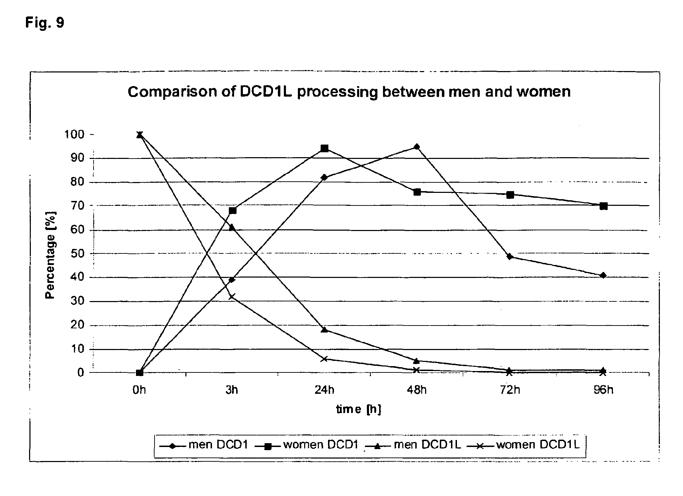

To analyze potential differences in the DCD1L processing between men and women, the conversion of DCD1L to DCD1 of six male and six female volunteers was studied. 280 µg DCD1L dissolved in 200 µl buffer (300 mM NaCl, 10 mM NaH₂PO₄, pH 9) was applied onto the test persons forearm using the cardboard/ceramic ring construction explained earlier. After three hours of incubation the peptide was removed from skin and further incubated for several hours *in vitro.* The DCD1L processing was directly analyzed by HPLC-MS after removal from skin and also after additional *in vitro* incubation at timepoints 24h, 48h, 72h, 96h, and 120 hours. The HPLC-MS results demonstrated that women are able to convert DCD1 L faster than men, additionally showing higher stability over the time (Fig.9).

### References

1. Ulvatne, H., Antimicrobial peptides: potential use in skin infections. Am J Clin Dermatol, 2003. 4(9): p. 591-5.
2. Schroder, J.M. and J. Harder, Antimicrobial skin peptides and proteins. Cell Mol Life Sci, 2006. 63(4): p. 469-86.
3. Schittek, B., et al., Dermcidin: a novel human antibiotic peptide secreted by sweat glands. Nat Immunol, 2001. 2(12): p. 1133-7.
4. Garbe, C. and B. Schittek, Antimicrobially active peptide, E.P. Office, Editor. 2006.
5. Baechle, D., et al., Cathepsin D is present in human eccrine sweat and involved in the postsecretory processing of the antimicrobial peptide DCD-1L. J Biol Chem, 2006. 281(9): p. 5406-15.
6. Steffen, H., et al., Naturally processed dermcidin-derived peptides do not permeabilize bacterial membranes and kill microorganisms irrespective of their charge. Antimicrob Agents Chemother, 2006. 50(8): p. 2608-20.
7. Lai, Y.P., et al., Functional and structural characterization of recombinant dermcidin-1L, a human antimicrobial peptide. Biochem Biophys Res Commun, 2005. 328(1): p. 243-50.
8. Cipakova, I., J. Gasperik, and E. Hostinova, Expression and purification of human antimicrobial peptide, dermcidin, in Escherichia coli. Protein Expr Purif, 2006. 45(2): p. 269-74.
9. Laemmli, U.K., Cleavage of structural proteins during the assembly of the head of bacteriophage T4. Nature, 1970. 227(5259): p. 680-5.
10. Kyhse-Andersen, J., Electroblotting of multiple gels: a simple apparatus without buffer tank for rapid transfer of proteins from polyacrylamide to nitrocellulose. J Biochem Biophys Methods, 1984. 10(3-4): p. 203-9.
11. B. Gasser et al., Transcriptomics-based identification of novel factors enhancing heterologous protein secretion in yeasts. Applied and Environmental Microbiology 2007, 73, 20:p. 6499.

## Claims

1. A method of producing a peptide consisting of the amino acids 63 to 110 of dermcidin according to SEQ ID NO: 3 without sequences extending beyond said peptide comprising
(a) culturing a host cell carrying a nucleic acid molecule encoding the peptide without sequences extending beyond the peptide in an expressible form, and
(b) optionally isolating the peptide from the culture,
wherein the host cell is a prokaryote cell or a fungal/yeast cell.

2. The method of claim 1, wherein at least 50 milligrams peptide per litre of cultured host cells are produced.

3. The method of claim 1 or 2 further comprising
(c) subjecting the peptide of claim 1(b) to proteolytic cleavage with an carboxypeptidase which cleaves off the C-terminal leucine in amino acid position 110 of dermcidin, and
(d) isolating the peptide from the resulting cleavage product.

4. The method of any of claims 1 to 3 further comprising purifying the peptide to homogeneity.

5. The method of claim 3 or 4 further comprising the step of formulating the peptide with a pharmaceutically acceptable carrier, diluent or excipient.

6. The method of any one of claims 1 to 5 further comprising the step of admixing the peptide to a skin benefit agent or dermatological benefit agent.

7. The method of claim 5 or 6 further comprising packaging the product obtained in unit dosage form.

## Patentansprüche

1. Verfahren zur Herstellung eines Peptids, das aus den Aminosäuren 63 bis 110 von Dermcidin gemäß SEQ ID NO:3 ohne Sequenzen, die dieses Peptid verlängern, besteht, umfassend:
(a) Züchtung einer Wirtszelle, die ein Nukleinsäuremolekül trägt, das das Peptid ohne Sequenzen, die dieses Peptid verlängern, in einer exprimierbaren Form kodiert, und
(b) gegebenenfalls Isolierung des Peptids aus der Kultur,
wobei die Wirtszelle eine prokaryotische oder eine Pilz-/Hefe-Zelle ist.

2. Verfahren nach Anspruch 1, wobei mindestens 50 Milligramm Peptid pro Liter gezüchteter Zellen hergestellt werden.

3. Verfahren nach Anspruch 1 oder 2 weiterhin umfassend:
(c) proteolytische Spaltung des Peptids nach Anspruch 1 (b) mit einer Carboxypeptidase, die das C-terminale Leucin in der Aminosäureposition 110 des Dermcidins abspaltet, und
(d) Isolierung des Peptids aus dem sich ergebenden Spaltprodukt.

4. Verfahren nach einem der Ansprüche 1 bis 3 weiterhin umfassend die Aufreinigung des Peptids bis zur Homogenität.

5. Verfahren nach Anspruch 3 oder 4 weiterhin umfassend den Schritt der Formulierung des Peptids mit einem pharmazeutisch verträglichen Träger, Verdünnungsmittel oder Exzipienten.

6. Verfahren nach einem der Ansprüche 1 bis 5 weiterhin umfassend den Schritt des Beimischens des Peptids zu einem günstig auf die Haut wirkenden Agens oder zu einem dermatologisch günstig wirkenden Agens.

7. Verfahren nach Anspruch 5 oder 6 weiterhin umfassend die Verpackung des erhaltenen Produkts in eine Einheits-Darreichungs-Form.

## Revendications

1. Méthode pour produire un peptide constitué des acides aminés 63 à 110 de la dermcidine selon SEQ ID NO: 3 sans séquences s'étendant au-delà dudit peptide comprenant
(a) la culture d'une cellule hôte portant une molécule d'acide nucléique codant pour le peptide sans séquences s'étendant au-delà du peptide dans une forme exprimable, et
(b) éventuellement, l'isolement du peptide à partir de la culture,
la cellule hôte étant une cellule procaryote ou une cellule fongique/de levure.

2. Méthode selon la revendication 1, dans laquelle sont produits au moins 50 milligrammes du peptide par litre de cellules hôtes cultivées.

3. Méthode selon la revendication 1 ou 2 comprenant en outre
(c) la soumission du peptide de la revendication 1(b) à un clivage protéolytique au moyen d'une carboxypeptidase qui clive la leucine C-terminale à la position d'acide aminé 110 de la dermcidine, et
(d) l'isolement du peptide à partir du produit de clivage résultant.

4. Méthode selon l'une quelconque des revendications 1 à 3 comprenant en outre la purification du peptide jusqu'à homogénéité.

5. Méthode selon la revendication 3 ou 4 comprenant en outre l'étape de formulation du peptide avec un support, diluant ou excipient pharmaceutiquement acceptable.

6. Méthode selon l'une quelconque des revendications 1 à 5 comprenant en outre l'étape consistant à mélanger le peptide à un agent bénéfique pour la peau ou un agent bénéfique sur le plan dermatologique.

7. Méthode selon la revendication 5 ou 6 comprenant en outre le conditionnement du produit obtenu dans une forme posologique unitaire.


### SEQUENCE LISTING

<110> B.R.A.I.N. AG
<120> A novel method for the production of a antimicrobial peptide <130> P1201 PCT
<150> EP 09 01 2228.4
   <151> 2009-09-25
<160> 7
<170> PatentIn version 3.4
<210> 1
   <211> 110
   <212> PRT
   <213> homo sapiens
<400> 1
   Met Arg Phe Met Thr Leu Leu Phe Leu Thr Ala Leu Ala Gly Ala Leu
   1 5 10 15
   Val Cys Ala Tyr Asp Pro Glu Ala Ala Ser Ala Pro Gly Ser Gly Asn
   20 25 30
   Pro Cys His Glu Ala Ser Ala Ala Gln Lys Glu Asn Ala Gly Glu Asp
   35 40 45
   Pro Gly Leu Ala Arg Gln Ala Pro Lys Pro Arg Lys Gln Arg Ser Ser
   50 55 60
   Leu Leu Glu Lys Gly Leu Asp Gly Ala Lys Lys Ala Val Gly Gly Leu
   65 70 75 80
   Gly Lys Leu Gly Lys Asp Ala Val Glu Asp Leu Glu Ser Val Gly Lys
   85 90 95
   Gly Ala Val His Asp Val Lys Asp Val Leu Asp Ser Val Leu
   100 105 110
<210> 2
   <211> 47
   <212> PRT
   <213> homo sapiens
<400> 2 <210> 3
   <211> 48
   <212> PRT
   <213> homo sapiens
<400> 3
<210> 4
   <211> 25
   <212> PRT
   <213> homo sapiens
<400> 4
   Ser Ser Leu Leu Glu Lys Gly Leu Asp Gly Ala Lys Lys Ala Val Gly
   1               5                   10                  15
   Gly Leu Gly Lys Leu Gly Lys Asp Ala
               20                  25
<210> 5
   <211> 23
   <212> PRT
   <213> homo sapiens
<400> 5
   Ser Ser Leu Leu Glu Lys Gly Leu Asp Gly Ala Lys Lys Ala Val Gly
   1               5                   10                  15
   Gly Leu Gly Lys Leu Gly Lys
               20
<210> 6
   <211> 90
   <212> PRT
   <213> homo sapiens
<400> 6
   Tyr Asp Pro Glu Ala Ala Ser Ala Pro Gly Ser Gly Asn Pro Cys His
   1               5                   10                  15
   Glu Ala Ser Ala Ala Gln Lys Glu Asn Ala Gly Glu Asp Pro Gly Leu
                   20                  25                  30
   Ala Arg Gln Ala Pro Lys Pro Arg Lys Gln Arg Ser Ser Leu Leu Glu
           35                  40                  45
   Lys Gly Leu Asp Gly Ala Lys Lys Ala Val Gly Gly Leu Gly Lys Leu
       50                  55                  60
   Gly Lys Asp Ala Val Glu Asp Leu Glu Ser Val Gly Lys Gly Ala Val
   65                  70                  75                  80
   His Asp Val Lys Asp Val Leu Asp Ser Val
                   85                  90
<210> 7
   <211> 91
   <212> PRT
   <213> homo sapiens
<400> 7
   Tyr Asp Pro Glu Ala Ala Ser Ala Pro Gly Ser Gly Asn Pro Cys His
   1               5                   10                  15
   Glu Ala Ser Ala Ala Gln Lys Glu Asn Ala Gly Glu Asp Pro Gly Leu
               20                  25                  30
   Ala Arg Gln Ala Pro Lys Pro Arg Lys Gln Arg Ser Ser Leu Leu Glu
           35                  40                  45
   Lys Gly Leu Asp Gly Ala Lys Lys Ala Val Gly Gly Leu Gly Lys Leu
       50                  55                  60
   Gly Lys Asp Ala Val Glu Asp Leu Glu Ser Val Gly Lys Gly Ala Val
   65                  70                  75                  80
   His Asp Val Lys Asp Val Leu Asp Ser Val Leu
                   85                  90